# FASCICULE DE BREVET EUROPEEN

(11) **EP 1 073 374 B1**
(45) Date de publication et mention de la délivrance du brevet: **30.03.2005**
(21) Numéro de dépôt: 99914634.3
(22) Date de dépôt: 21.04.1999
(51) Int. Cl.: A61B 17/04, F16B 13/06

(54) **ANCRE DE SUTURE A EXPANSION REVERSIBLE**
WIEDERLÖSBARER SPREIZANKER FÜR CHIRURGISCHEN NÄHFADEN
SUTURE ANCHOR WITH REVERSIBLE EXPANSION

(30) Priorité: 21.04.1998 FR 9805203
(43) Date de publication de la demande: 07.02.2001
(60) Demande divisionnaire: 04029007.4
(73) Titulaire: Tornier SA, 38330 Saint Ismier (FR)
(72) Inventeur: TORNIER, Alain, F-38330 Saint Ismier (FR); BONNOMET, François, F-67000 Strasbourg (FR)
(74) Mandataire: Schmitt, John
(86) Numéro de dépôt international: PCT/FR1999/000941
(87) Numéro de publication internationale: WO 1999/053844

(56) Documents cités:
- GB-A- 2 173 565
- US-A- 5 472 452
- US-A- 5 501 695
- US-A- 5 649 963

## Description

La présente invention est relative à une ancre de suture permettant de rattacher des tissus mous tels que des ligaments ou tendons à l'os, et principalement pour la réparation de la coiffe des rotateurs et des lésions de Bankart.

On connaît d'après le brevet américain n° 5 501 695 au nom de Anspach une ancre de suture en deux parties permettant la réinsertion musculaire et tendineuse sur l'os.

Cette ancre de suture comprend un premier élément extérieur cylindrique solidaire de branches de fixation qui sont séparées les unes des autres par des fentes disposées parallèlement à l'axe longitudinal de l'ancre de suture. Le premier élément reçoit dans sa partie interne un second élément de déformation qui coopère avec l'extrémité libre des branches de fixation.

Le second élément de déformation est solidaire par l'intermédiaire d'une zone de rupture d'une tige de traction qui permet au chirurgien, après avoir introduit l'ancre de suture dans un trou préalablement ménagé dans l'os, de faire coulisser ledit second élément à l'intérieur du premier afin de déformer axialement les branches de fixation dans la partie de l'os spongieux.

Lorsque l'effort de traction est suffisant pour déformer les branches de fixation la tige se sépare du second élément par une rupture irréversible.

On note que les branches se déforment latéralement suivant une direction sensiblement perpendiculaire à l'axe longitudinal de l'ancre de suture pour fixer définitivement cette dernière à l'intérieur de l'os.

Enfin, le premier élément est solidaire à l'une de ses extrémités d'une collerette qui vient en appui contre l'os cortical et qui est percée d'un certain nombre de trous pour la fixation par le chirurgien de fils de suture.

L'ancre de suture décrite ci-dessus comporte certains inconvénients à savoir qu'elle n'est pas prévue pour être retirée de l'os sans provoquer une destruction complète de cette dernière et de l'os dans laquelle elle est fixée. En effet l'ancre de suture ne comporte aucun moyen de reprise permettant son retrait de l'os sans engager une détérioration de ce dernier.

En outre, l'ancre de suture ne comporte pas en dehors de la zone de rupture du second élément, des moyens limitant la course dudit élément pour éviter que les branches de fixation viennent à se rompre anormalement sous l'effort de traction.

On connaît également du brevet US 5 649 963, une ancre de suture et un outil sur lequel se visse une douille pour la fixation de ladite ancre.

L'ancre de suture comporte des moyens d'expansion qui s'écartent en direction de l'extérieur sous la pression de la douille et de l'outil. On note que l'outil présente un dispositif qui permet de rapprocher les moyens d'expansion pour l'extraction de l'ancre de suture.

On constate que l'ancre de suture ne comporte pas de moyens de butée limitant la déformation des moyens d'expansion lors de l'application de la pression de la douille et de l'outil.

On connaît d'après le document US 5 472 452, une ancre de suture comportant des moyens d'expansion à déformation élastique, et un dispositif de déformation guidé à l'intérieur du corps creux de ladite ancre pour déformer les moyens d'expansion. Le dispositif de déformation présente un profil particulier permettant sous un effort de traction de déformer les moyens d'expansion élastique afin qu'ils pénètrent à l'intérieur de l'os spongieux qui se trouve au voisinage du trou dans lequel est préalablement introduit l'ancre de suture. Le dispositif de déformation présente un profil particulier permettant aux moyens d'expansion d'être bloqués dans une position qui retient l'ancre de suture dans l'os spongieux. Lors d'un effort de poussée, le dispositif de déformation pénètre à l'intérieur du corps de l'ancre de suture pour déformer à nouveau les moyens d'expansion dans une direction identique à celle de l'effort de traction.

On connaît d'après le document GB 2 173 565 une vis chirurgicale constituée de plusieurs pièces coopérant intimement ensemble pour permettre la déformation de moyens d'expansion réalisés dans une matière plastique à haute densité. La vis chirurgicale comporte un corps creux et cylindrique traversé par un élément fileté pourvu d'une tête, d'un écrou de serrage coopérant avec l'élément fileté, et des moyens d'expansion prévus entre le corps cylindrique et la tête de l'écrou de serrage. Lors de l'entraînement en rotation de l'élément fileté, l'écrou pénètre à l'intérieur du corps creux afin de déformer les moyens d'expansion qui sont collés à la tête dudit écrou.

L'ancre de suture suivant la présente invention à pour objet d'être réversible pour permettre son extraction de l'os sans avoir à percer un trou à un diamètre plus grand que celui des branches déformées.

L'ancre de suture suivant la présente invention permettant la fixation de tissus mous contre un os, comporte un corps creux comprenant d'une part à l'une de ses extrémités une tête pourvue de moyens de fixation des tissus mous contre l'os par le passage d'au moins un fil de suture, et d'autre part des moyens d'expansion pour la fixation de l'ancre de suture dans l'os, lesdits moyens d'expansion étant constitués par au moins deux branches de fixation disposées parallèlement à l'axe longitudinal (XX') dudit corps avant déformation, et au moins deux butées, intercalées entre chaque branche de fixation, limitant la déformation plastique de ces dernières lors de l'application d'un effort extérieur de traction (T) pour la fixation de l'ancre de suture dans l'os afin que la déformation desdits moyens d'expansion soit réversible lors de l'application d'un autre effort extérieur de poussée (P) permettant le retrait de ladite ancre de suture de l'os.

L'ancre de suture suivant un mode de réalisation préféré de la présente invention comporte un corps creux comprenant à l'opposé de la tête une pointe à profil conique.

L'ancre de suture suivant un mode de réalisation préféré de la présente invention comporte une tête comprenant des moyens de fixation qui sont constitués de deux languettes opposées et parallèles à l'axe longitudinal (XX') du corps creux, avant déformation, afin de délimiter un espace oblong qui est traversé par au moins un fil de suture, ledit fil de suture étant serti sur la tête après déformation des languettes sous un autre effort de traction pour permettre la ligature des tissus mous sur l'ancre de suture.

L'ancre de suture suivant un mode de réalisation préféré de la présente invention comporte une tête comprenant des moyens de fixation qui sont constitués d'un disque disposé perpendiculairement à l'axe longitudinal (XX') du corps creux, ledit disque étant conformé pour venir bloquer les tissus mous contre l'os.

L'ancre de suture suivant un mode de réalisation préféré de la présente invention comporte un corps creux comprenant dans la pointe à profil conique un trou borgne fileté qui est prévu pour recevoir une tige filetée d'un ancillaire pour déformer, d'une part sous un premier effort de traction (T) les branches de fixation suivant une direction sensiblement perpendiculaire à l'axe longitudinal (XX') dudit corps et d'autre part sous un second effort de traction (T1, T2) supérieur au premier (T) les moyens de fixation.

L'ancre de suture suivant un mode de réalisation préféré de la présente invention comporte un corps creux comprenant à proximité de la tête un alésage interne fileté qui est destiné à recevoir une première tige filetée creuse d'un ancillaire, tandis qu'une seconde tige coulissant dans la première tige vient en appui dans le fond d'un trou borgne fileté pour déplier sous un effort de poussée (P) les branches de fixation dans une position sensiblement allongée pour pouvoir extraire l'ancre de suture de l'os.

L'ancre de suture suivant un mode de réalisation préféré de la présente invention comporte des branches de fixation qui sont raccordées à une partie cylindrique de la tête et à la pointe à profil conique par des premières amorces de pliage dirigées en direction du centre du corps.

L'ancre de suture suivant un mode de réalisation préféré de la présente invention comporte des branches de fixation qui présentent respectivement en leur milieu une seconde amorce de pliage qui est inversée par rapport aux premières amorces de pliage de manière que chaque branche soit constituée de deux segments identiques.

L'ancre de suture suivant un mode de réalisation préféré de la présente invention comporte un alésage interne fileté et un trou borgne fileté qui sont portés par le même axe longitudinal (XX') du corps et qui sont prévus de diamètres différents.

L'ancre de suture suivant un mode de réalisation préféré de la présente invention comporte des butées qui présentent une longueur dépendant de la déformation que l'on désire obtenir des branches de fixation.

L'ancre de suture suivant un mode de la réalisation préféré de la présente invention comporte une pointe à profil conique présentant une face qui est disposée perpendiculairement à l'axe (XX').

L'ancre de suture suivant un mode de la réalisation préféré de la présente invention comporte des moyens de fixation dont l'espace oblong est positionné de manière que sa direction de plus grande longueur soit perpendiculaire à l'axe longitudinal (XX') du corps.

L'ancre de suture suivant un mode de la réalisation préféré de la présente invention comporte une tête comprenant une partie cylindrique pourvue sur sa périphérie externe d'une collerette circulaire formant une butée d'appui du corps contre la paroi externe de l'os.

L'ancre de suture suivant un mode de réalisation préféré de la présente invention comporte une tête présentant une partie cylindrique et une partie filetée d'un alésage interne qui est disposée à proximité du disque.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue illustrant l'ancre de suture suivant la présente invention.
Figure 2 est une vue semblable à celle de figure 1, mais montrant une variante de l'ancre de suture suivant la présente invention.
Figure 3 est une vue représentant une autre variante de l'ancre de suture suivant la présente invention.
Figure 4a à 4d sont des vues schématiques illustrant la mise en place de l'ancre de suture suivant figure 1 et 2 dans un os pour la réinsertion des tissus mous.
Figure 5a à 5d sont des vues schématiques montrant la mise en place de l'ancre de suture suivant figure 3.
Figure 6a à 6d sont des vues schématiques représentant l'extraction de l'ancre de suture de l'os.

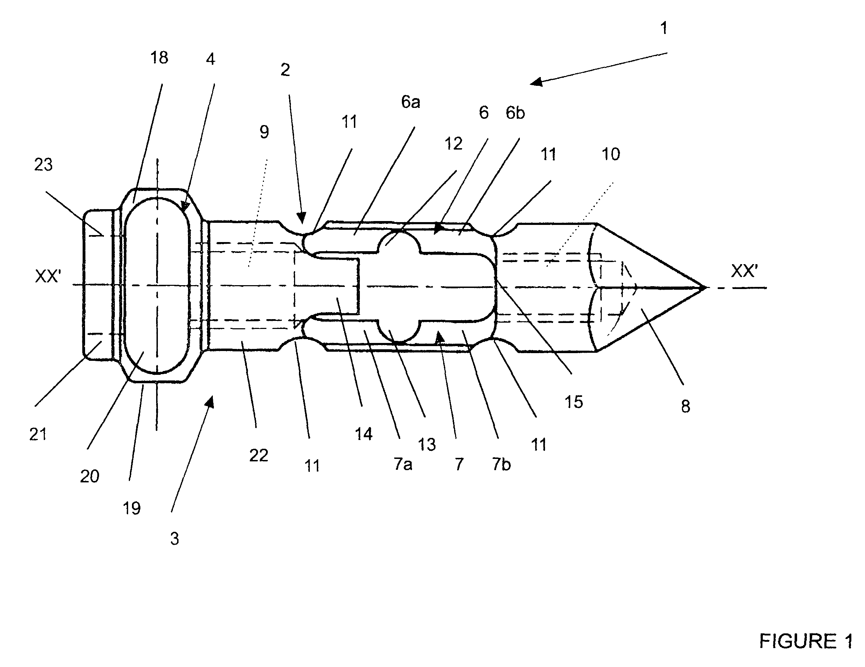

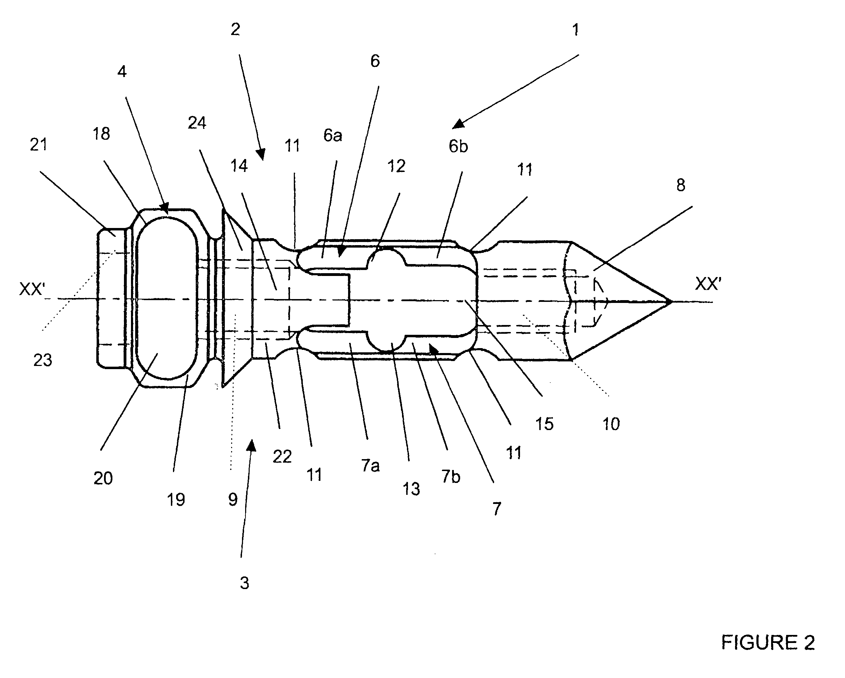

On a montré en figures 1 et 2 une ancre de suture monobloc 1 comportant un corps cylindrique creux 2 de forme allongée susceptible de se déformer plastiquement et pouvant être mis en place sur le site opératoire sous arthroscopie.

Le corps 2 est constitué à l'une de ses extrémités d'une tête 3 comportant des moyens de fixation 4 des tissus mous 16 contre l'os 17 d'un patient par l'intermédiaire d'un ou plusieurs fils de suture 5 sertis sur ladite tête.

La tête 3 est constituée des moyens de fixation 4 qui sont prévus entre une face cylindrique d'appui 21 et une partie cylindrique 22.

La partie cylindrique 22 de la tête 3 se prolonge à l'opposée des moyens de fixation 4 par au moins deux branches de fixation 6, 7 qui sont, avant déformation, parallèles à l'axe longitudinal XX' du corps 2.

L'autre extrémité du corps 2, se trouvant à l'opposée de celle de la tête 3, est constituée dans le prolongement des branches 6 et 7 d'une pointe à profil conique 8 facilitant la mise en place de l'ancre de suture dans l'os 17.

La partie cylindrique 22 de la tête 3 comporte un alésage interne fileté 9 qui débouche d'une part du coté des moyens de fixation 4 et d'autre part entre les branches de fixation 6 et 7. L'alésage fileté 9 est porté par l'axe longitudinal XX' du corps 2.

Également la pointe à profil conique 8 présente dans sa partie interne un trou borgne fileté 10 qui débouche entre les branches de fixation 6 et 7 et qui est porté par le même axe longitudinal XX' que celui de l'alésage 9. En Outre, le diamètre de l'alésage fileté 9 est prévu plus grand que celui du trou fileté 10.

Les branches 6 et 7 sont raccordées à la tête 3 et à la pointe 8 par des amorces de pliage 11 dirigées en direction du centre du corps 2 et qui permettront de déformer lesdites branches sous un effort de traction.

Les branches 6 et 7 présentent respectivement en leur milieu une amorce de pliage 12, 13 qui est inversée par rapport à celles 11 de manière que chaque branche soit constituée de deux segments identiques 6a, 6b et 7a, 7b.

Entre chaque branche 6 et 7 sont prévus des moyens de butée 14 solidaire de la partie cylindrique 22 de la tête 3 et qui est dirigée en direction de la pointe à profil conique 8. Chaque butée 14 s'étend parallèlement à l'axe longitudinal XX' du corps 2 et présente une longueur qui dépend de la déformation que l'on désire obtenir des branches 6 et 7.

En effet, la déformation plastique des branches 6 et 7 est limitée par les butées 14 qui viennent en appui contre une face 15 de la pointe à profil conique 8. La face 15 est disposée dans un plan perpendiculaire à celui portant l'axe XX' du corps 2.

Les moyens de fixation 4 sont constitués de deux languettes 18, 19 opposées et parallèles à l'axe longitudinal XX' du corps 2, avant leur déformation. Les languettes 18 et 19 délimitent un espace oblong 20 qui permet le passage, par le chirurgien sur le site opératoire, d'au moins un fil de suture 5.

L'espace oblong 20, des moyens de fixation 4, est positionné de manière que sa direction de plus grande longueur soit perpendiculaire à l'axe longitudinal XX' du corps 2 de l'ancre de suture 1.

La face d'appui 21, réunissant les languettes 18, 19 à l'opposée de la partie cylindrique 22, est percée d'un alésage 23, coaxial à celui fileté 9, et qui débouche d'une part à l'extérieur du corps 2 et d'autre part entre lesdites languettes pour être positionné en face dudit alésage fileté 9 de ladite partie cylindrique 22.

La partie cylindrique 22 de la tête 3 comporte sur sa périphérie externe et entre les moyens de fixation 4 et les branches 6, 7 une collerette circulaire 24 formant une butée d'appui du corps 2 contre la paroi externe de l'os 17 lors de l'introduction de l'ancre de suture 1 à l'intérieur de ce dernier (figure 2). La collerette 24 présente un profil conique qui vient en contact avec l'os 17 pour servir de butée lors de l'effondrement des branches de fixation 6 et 7.

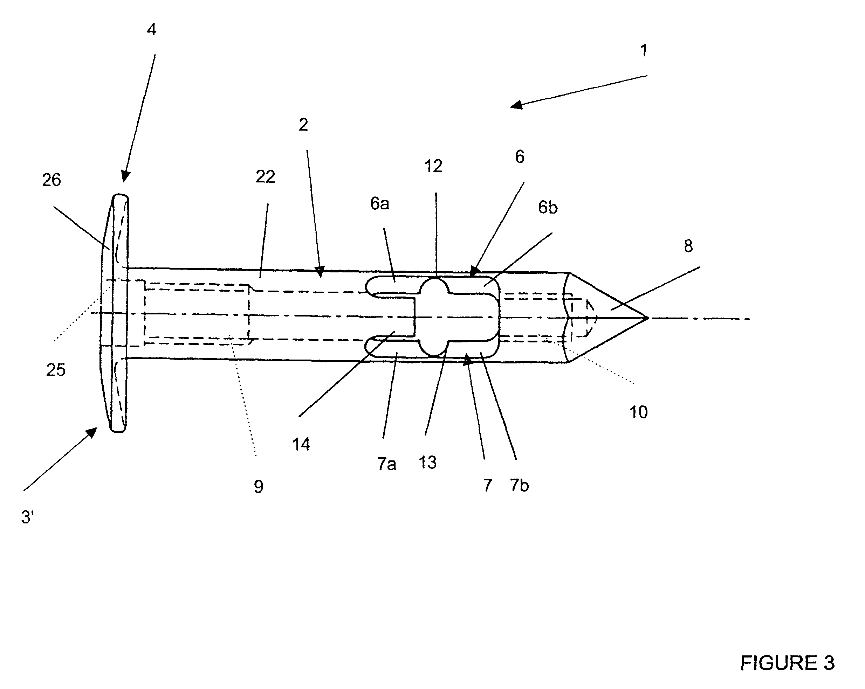

En figure 3 on a représenté une variante de l'ancre de suture 1 dont le corps cylindrique 2 présente une tête 3' différente de celle 3 décrite précédemment en ce qui concerne les moyens de fixation 4 des tissus mous.

La tête 3' est constituée d'une partie cylindrique 22 plus longue que celle décrite précédemment et dans laquelle est percée l'alésage interne et fileté 9. Ce dernier débouche d'une part entre les branches 6, 7 et d'autre part à l'extérieur du corps 2 au niveau des moyens de fixation 4 par l'intermédiaire d'un alésage coaxial 25.

L'alésage interne 9 présente sur sa longueur une partie filetée qui est plus courte que celle montrée en figures 1 et 2. De plus, on remarque dans cette variante que la partie filetée de l'alésage interne 9 est éloignée des butées 14 pour se trouver à proximité des moyens de fixation 4 du fait de la partie cylindrique 22 plus longue.

Les moyens de fixation 4 sont constitués à l'extrémité de la partie cylindrique 22 de la tête 3' et à l'opposé des branches 6,7 par un disque 26 qui peut être conformé pour permettre la retenue des tissus mous 16 contre l'os 17.

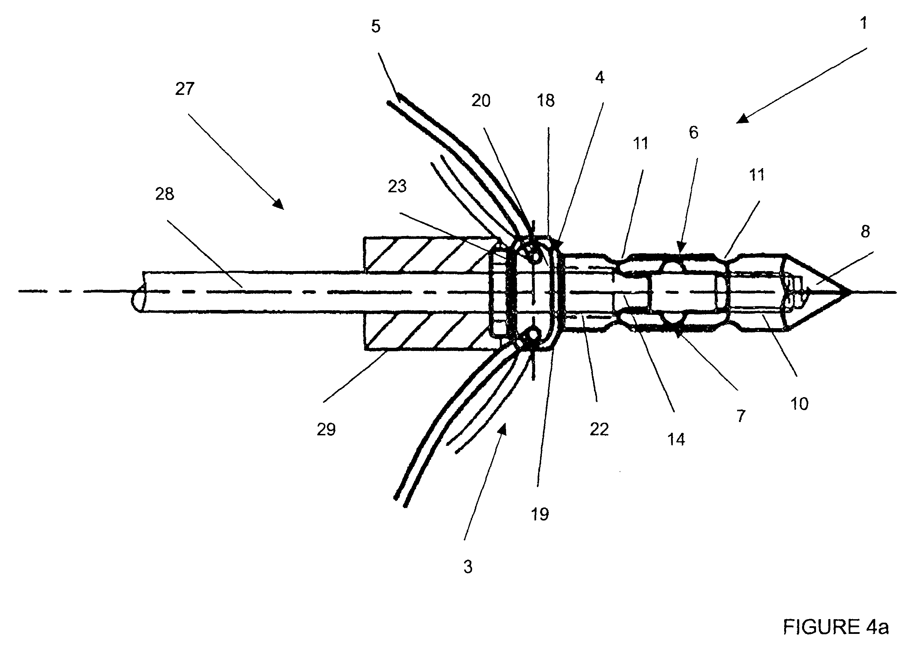

On a représenté en figures 4a à 4d les différentes étapes pour la mise en place de l'ancre de suture 1 décrite précédemment à l'intérieur de l'os 17 pour la fixation des tissus mous 16.

La figure 4a montre l'ancre de suture 1 munie de son ancillaire 27 de mise en place qui est constitué, par exemple, d'une tige 28 qui traverse le corps 2 pour venir se visser dans le trou borgne 10 de la pointe à profil conique 8. La tige 28 est solidaire d'un embout 29 qui vient prendre appui contre la face 23 de la tête 3.

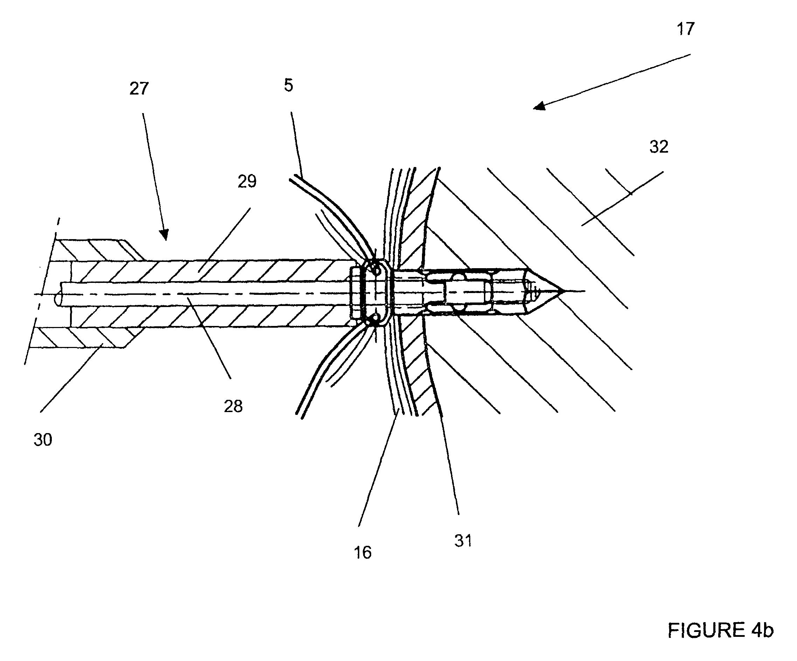

La figure 4b représente l'ancre de suture 1 qui est introduite dans le site opératoire par l'intermédiaire de l'ancillaire 27 et d'une canule d'arthroscopie 30. Le chirurgien procède à la mise en place des fils de suture 5 dans l'espace oblong 20 des moyens de fixation 4, soit avant l'introduction de l'ancre de suture 1 dans le site opératoire, soit après sa mise en place dans l'os 17.

La mise en place de l'ancre de suture 1 dans l'os 17 est réalisée soit par force, soit par rotation, soit par l'intermédiaire d'un pré-trou percé dans l'os cortical 31 et l'os spongieux 32, à travers le tissu mou 16 à réinsérer.

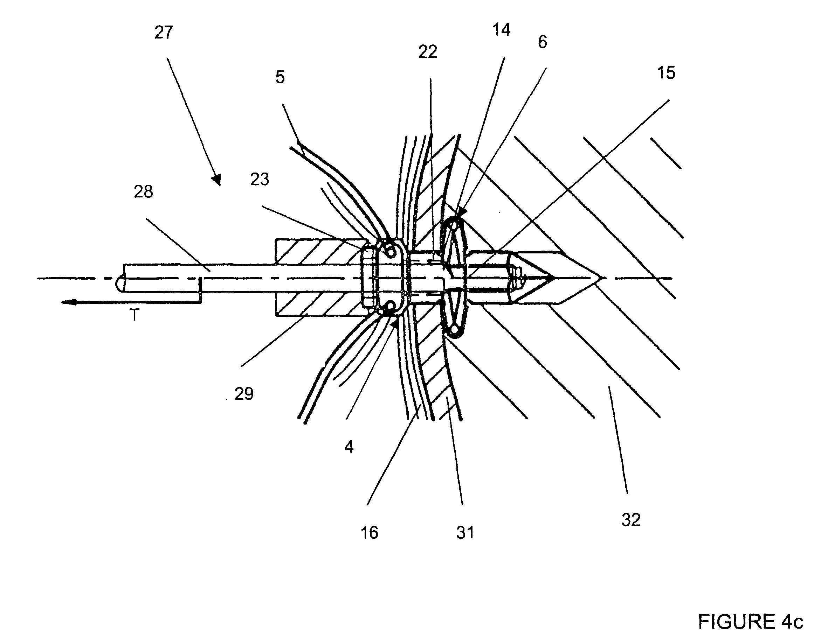

La figure 4c montre la déformation de l'ancre de suture 1 et plus particulièrement des branches 6 et 7 à l'intérieur de l'os spongieux 32 lorsqu'un effort de traction T est soumis à la tige 28 de l'ancillaire 27. Ainsi la tige 28 se déplace horizontalement suivant l'axe XX' du corps 2, tandis que l'embout 29 reste fixe en appui contre la face 23 de la tête 3.

La déformation des branches 6 et 7 est limitée jusqu'à ce que la pointe à profil conique 8 vienne par l'intermédiaire de sa paroi 15 en appui contre les butées 14.

Les branches 6 et 7 se déforment, sous un effort de compression du fait de la traction T soumise à la tige 28 de l'ancillaire 27, suivant le profil des amorces 11, 12 et 13 de manière que les segments 6a, 6b et 7a, 7b soient dirigés à l'extérieur du corps 2 et dans une direction sensiblement perpendiculaire à l'axe XX'.

On note que la fixation de l'ancre de suture 1 dans l'os spongieux 32 est réalisée par la déformation des branches 6 et 7 jusqu'à ce que les segments 6a et 7a viennent en contact avec la face interne de l'os cortical 31.

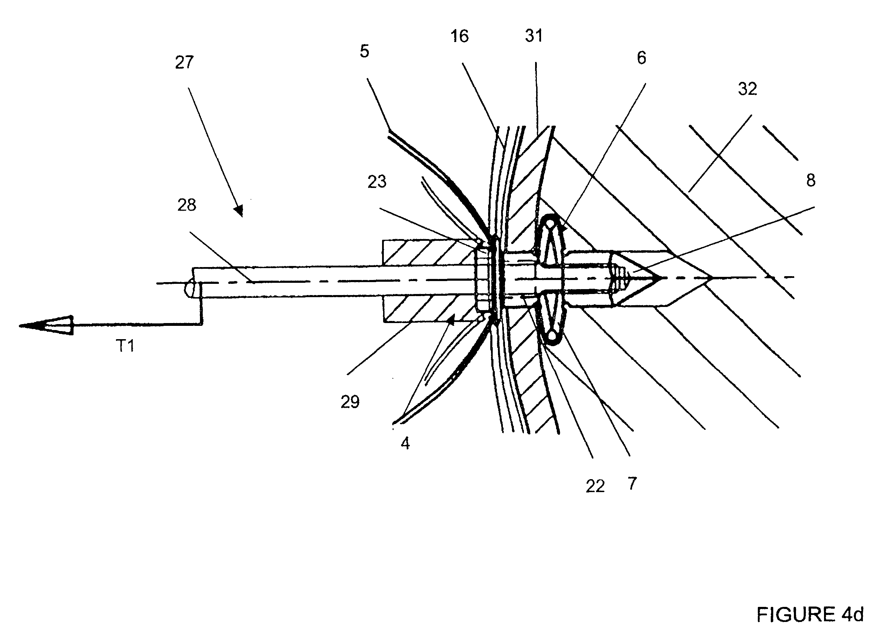

La figure 4d illustre le sertissage des fils de suture 5 sur la tête 3 de l'ancre 1 par l'intermédiaire des moyens de fixation 4.

La pointe à profil conique 8 étant en contact avec les butées 14, il est possible d'appliquer un effort de traction T1 plus important que celui T sur la tige 28 de l'ancillaire 27, sans risquer de rompre les branches 6 et 7, afin de déformer les languettes 18 et 19 des moyens de fixation 4.

La déformation des languettes 18 et 19 permet de réduire l'espace oblong 20 de manière à bloquer dans une position tendue les fils de suture 5 sur la tête 3 de l'ancre de suture 1.

Les languettes 18 et 19 sont conformées pour ne pas couper les fils de suture 5 lors du sertissage de ces derniers sur la tête 3.

Les fils de suture 5 permettent aux chirurgiens de ligaturer les tissus mous 16 sur l'ancre de suture 1.

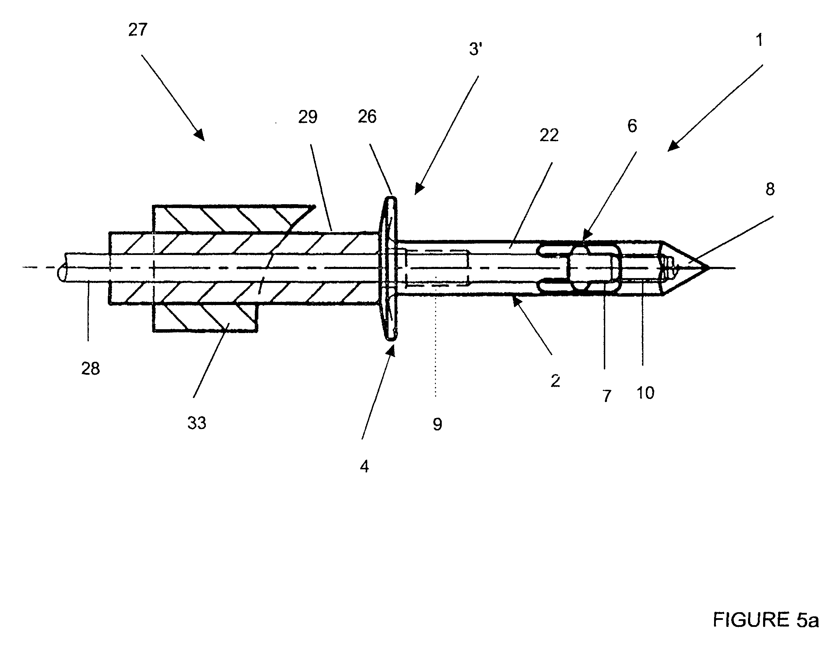

On a représenté en figures 5a à 5d les différentes étapes pour la mise en place de l'ancre de suture 1 décrite précédemment en figure 3 à l'intérieur de l'os 17 pour la fixation des tissus mous 16.

La figure 5a montre l'ancre de suture 1 munie de son ancillaire 27 de mise en place qui est constitué de la tige 28 qui traverse le corps 2 pour venir se visser dans le trou borgne 10 de la pointe à profil conique 8. La tige 28 est solidaire d'un embout 29 qui vient prendre appui contre le disque 26 de la tête 3'.

Autour de l'embout 29 est prévu un outil de mise en forme 33 du disque 26 sur le tissu mou 16.

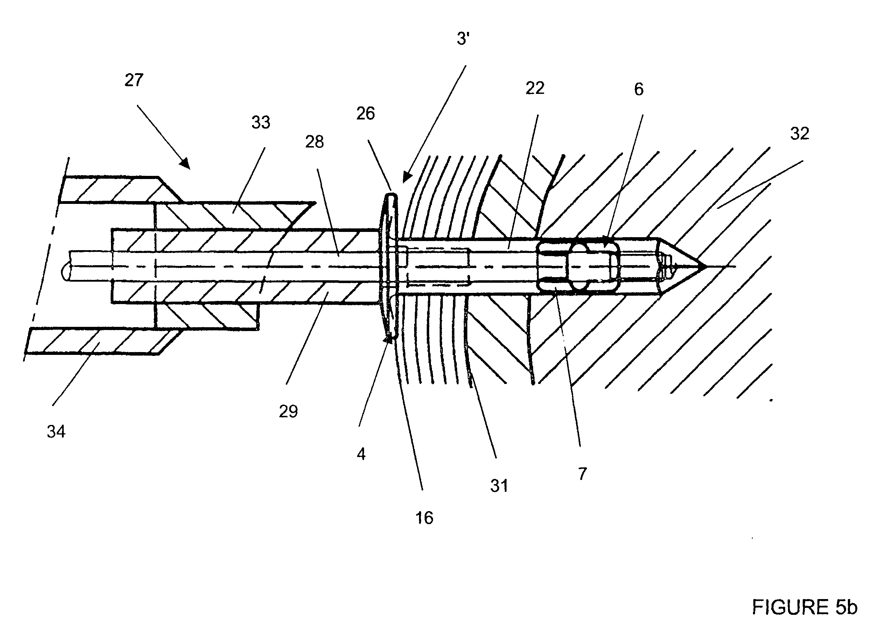

La figure 5b représente l'ancre de suture 1 qui est introduite dans le site opératoire par l'intermédiaire de l'ancillaire 27 et d'une canule d'arthroscopie 34.

La mise en place de l'ancre de suture 1 dans l'os 17 est réalisée soit par force, soit par rotation, soit par l'intermédiaire d'un pré-trou percé dans l'os cortical 31 et l'os spongieux 32, à travers le tissu mou 16 à réinsérer.

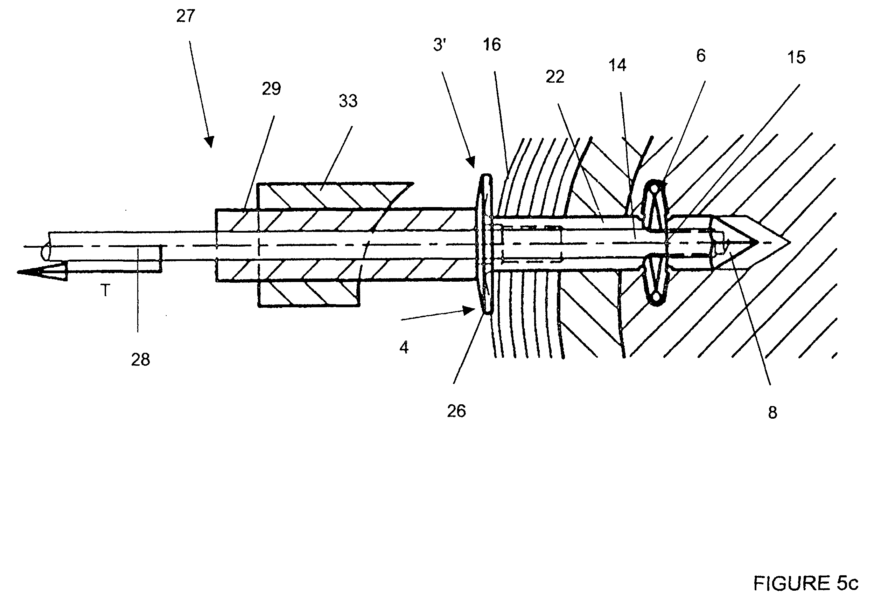

La figure 5c montre la déformation de l'ancre de suture 1 et plus particulièrement des branches 6 et 7 à l'intérieur de l'os spongieux 32 lorsqu'un effort de traction T est soumis à la tige 28 de l'ancillaire 27. Ainsi la tige 28 se déplace horizontalement suivant l'axe XX' du corps 2, tandis que l'embout 29 reste fixe en appui contre le disque 26 de la tête 3'.

La déformation des branches 6 et 7 est limitée jusqu'à ce que la pointe à profil conique 8 vienne par l'intermédiaire de sa face 15 en appui contre les butées 14.

Les branches 6 et 7 se déforment, sous un effort de compression du fait de la traction T soumise à la tige 28 de l'ancillaire 27, suivant le profil des amorces 11, 12 et 13 de manière que les segments 6a, 6b et 7a, 7b soient dirigés à l'extérieur du corps 2 et dans une direction sensiblement perpendiculaire à l'axe XX'.

On note que la fixation de l'ancre de suture 1 dans l'os spongieux 32 est réalisée par la déformation des branches 6 et 7 jusqu'à ce que les segments 6a et 7a viennent en contact avec la face interne de l'os cortical 31.

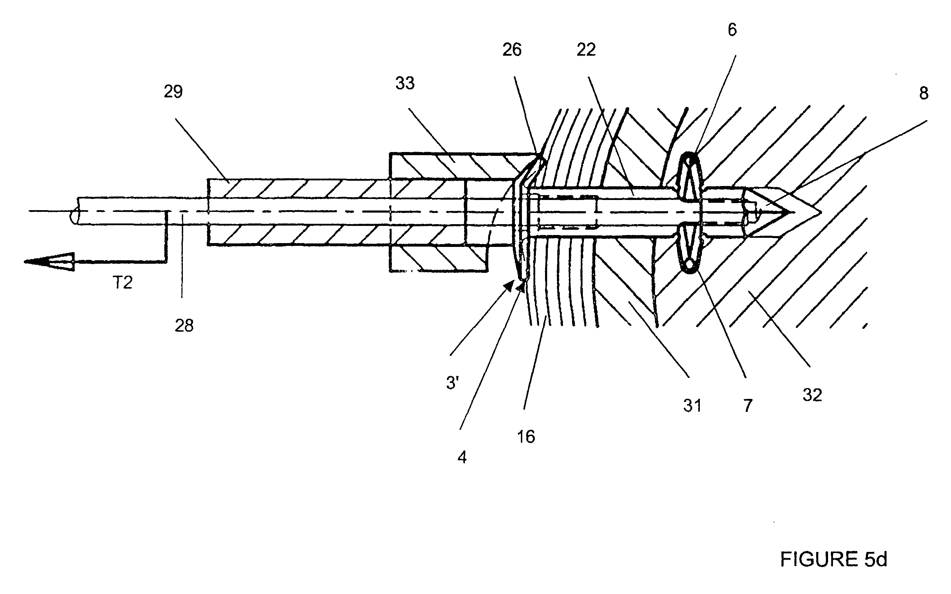

En figure 5d on a montré la conformation du disque 26 par l'outil 33, après retrait préalable de l'embout 29, afin d'appliquer un nouvel effort de traction T2 supérieur à celui T permettant la déformation des branches 6 et 7.

L'effort T2 permet au chirurgien de plier le disque 26 de la tête 3' pour qu'il pénètre dans le tissu mou 16 afin de le plaquer contre l'os cortical 31.

La conformation ou le pliage du disque 26 est réalisé en plusieurs fois. Il suffit au chirurgien de dévisser la tige 28 pour positionner l'outil 33 suivant une autre direction, de revisser la tige 28 dans le trou 10 de la pointe 8 et d'appliquer de nouveau l'effort de traction T2 pour plier le disque 26.

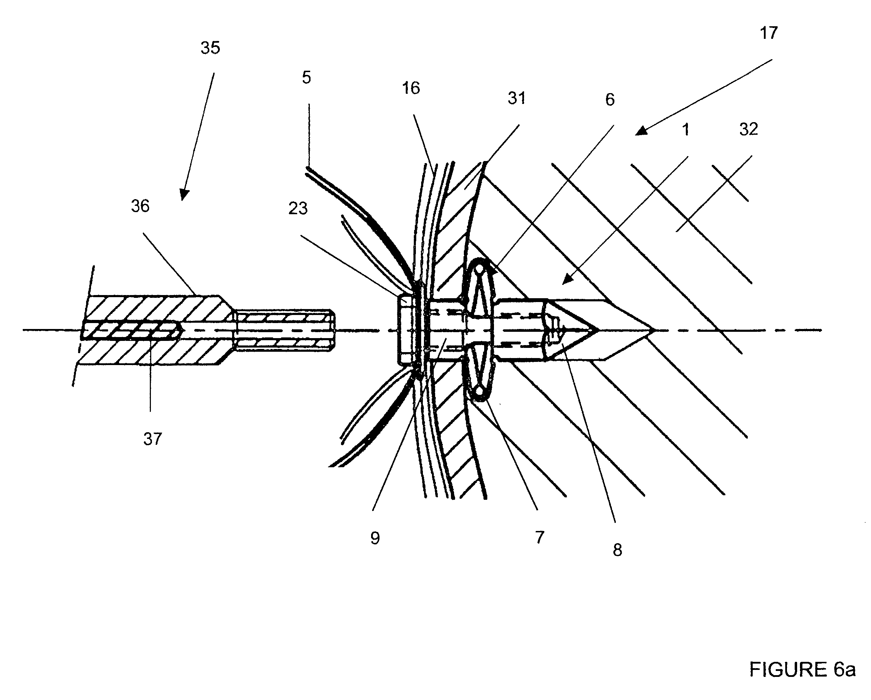

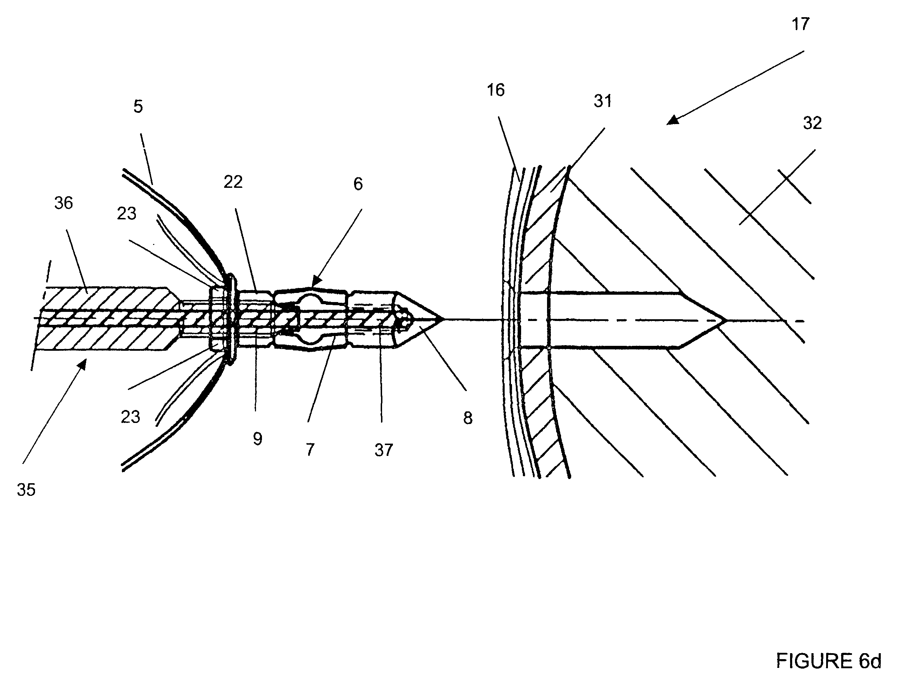

En figures 6a à 6d on a illustré les différentes étapes pour extraire l'ancre de suture 1 de l'os 17 au moyen d'un autre ancillaire 35. Ce dernier permet l'extraction de l'ancre de suture 1 comportant la tête 3 ou 3'.

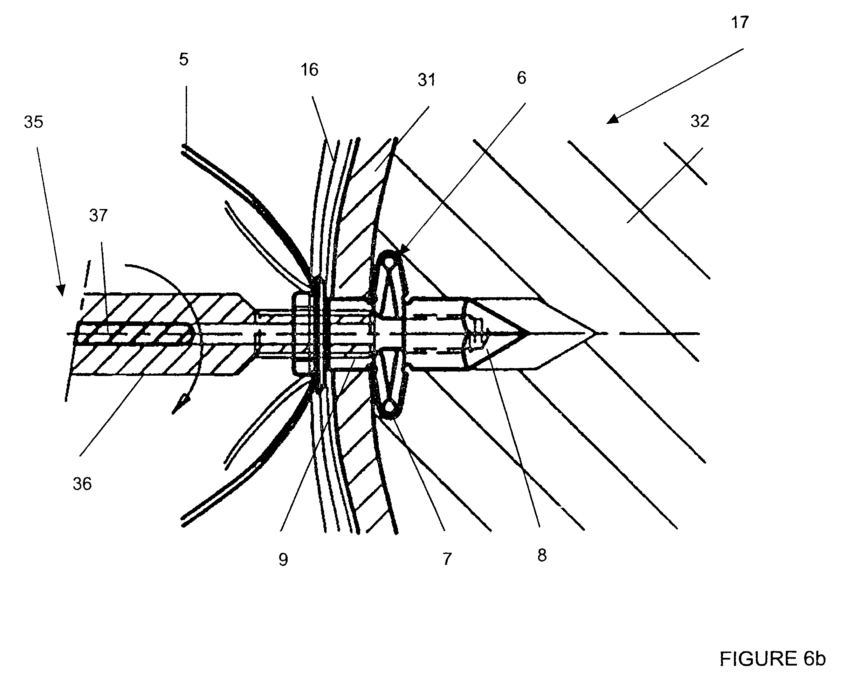

L'ancillaire 35 comporte une tige creuse 36 qui vient se visser dans l'alésage fileté 9 de la partie cylindrique 22, tandis qu'une autre tige 37 coulissant dans la première vient prendre appui dans le fond du trou borgne 10 ménagé dans la pointe à profil conique 8 (figure 6b).

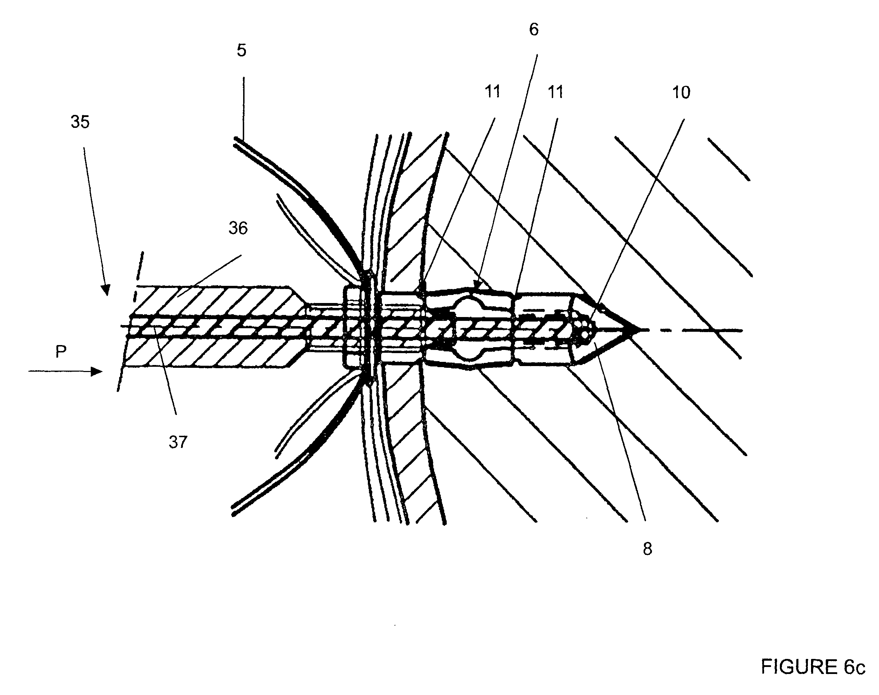

La tige 36 est soumise à un effort de poussée P parallèle à l'axe XX' du corps 2, afin de déplier les branches 6 et 7 (figure 6c). Le profil des amorces 11, 12 et 13 permet de ramener l'ancre de suture 1 suivant une forme semblable à celle d'origine.

Dès que l'ancre de suture 1 a retrouvé une position allongée, le chirurgien peut, à l'aide de l'ancillaire 35, retirer ladite ancre de l'os 17, sans à avoir à percer un trou dont le diamètre est sensiblement voisin de celui affecter par les branches déformées.

## Revendications

1. Ancre de suture permettant la fixation de tissus mous (16) contre un os (17), comportant un corps creux (2) comprenant d'une part à l'une de ses extrémités une tête (3, 3') pourvue de moyens de fixation (4) des tissus mous (16) contre l'os (17) par le passage d'au moins un fil de suture (5), et d'autre part des moyens d'expansion pour la fixation de l'ancre de suture dans l'os (17), les dits moyens d'expansion étant constitués par au moins deux branches de fixation (6, 7) disposées parallèlement à l'axe longitudinal (XX') dudit corps creux avant déformation, et au moins deux butées (14), intercalées entre chaque branche de fixation (6, 7), limitant la déformation plastique de ces dernières lors de l'application d'un effort extérieur de traction (T) pour la fixation de l'ancre de suture (1) dans l'os (17) afin que la déformation desdits moyens d'expansion (6, 7) soit réversible lors de l'application d'un autre effort extérieur de poussée (P) permettant le retrait de ladite ancre de suture (1) de l'os (17).

2. Ancre de suture suivant la revendication 1, **caractérisée en ce que** le corps creux (2) comprend à l'opposé de la tête (3, 3') une pointe à profil conique (8).

3. Ancre de suture suivant la revendication 1, **caractérisée en ce que** les moyens de fixation (4) de la tête (3) sont constitués de deux languettes (18, 19) opposées et parallèles à l'axe longitudinal (XX') du corps creux (2), avant déformation, afin de délimiter un espace oblong (20) qui est traversé par au moins un fil de suture (5), ledit fil de suture (5) étant serti sur la tête (3) après déformation des languettes (18, 19) sous un autre effort de traction pour permettre la ligature des tissus mous (16) sur l'ancre de suture (1).

4. Ancre de suture suivant la revendication 1, **caractérisée en ce que** les moyens de fixation (4) de la tête (3') sont constitués d'un disque (26) disposé perpendiculairement à l'axe longitudinal (XX') du corps creux (2), ledit disque étant conformé pour venir bloquer les tissus mous (16) contre l'os (17).

5. Ancre de suture suivant la revendication 2, **caractérisée en ce que** le corps creux (2) comporte dans la pointe à profil conique (8) un trou borgne fileté (10) qui est prévu pour recevoir une tige filetée (28) d'un ancillaire (27) pour déformer, d'une part sous un premier effort de traction (T) les branches de fixation (6, 7) suivant une direction sensiblement perpendiculaire à l'axe longitudinal (XX') dudit corps et d'autre part sous un second effort de traction (T1, T2) supérieur au premier (T) les moyens de fixation (4).

6. Ancre de suture suivant la revendication 1, **caractérisée en ce que** le corps creux (2) comporte à proximité de la tête (3, 3') un alésage interne fileté (9) qui est destiné à recevoir une première tige filetée creuse (36) d'un ancillaire (35), tandis qu'une seconde tige (37) coulissant dans la première tige (36) vient en appui dans le fond d'un trou borgne fileté (10) pour déplier sous un effort de poussée (P) les branches de fixation (6, 7) dans une position sensiblement allongée pour pouvoir extraire l'ancre de suture (1) de l'os (17).

7. Ancre de suture suivant la revendication 2, **caractérisée en ce que** les branches de fixation (6, 7) sont raccordées à une partie cylindrique (22) de la tête (3, 3') et à la pointe à profil conique (8) par des premières amorces de pliage (11) dirigées en direction du centre du corps creux (2).

8. Ancre de suture suivant la revendication 6, **caractérisée en ce que** les branches de fixation (6, 7) présentent respectivement en leur milieu une seconde amorce de pliage (12, 13) qui est inversée par rapport aux premières amorces de pliage (11) de manière que chaque branche soit constituée de deux segments identiques (6a, 6b ; 7a, 7b).

9. Ancre de suture suivant la revendication 6, **caractérisée en ce que** l'alésage interne fileté (9) et le trou borgne fileté (10) sont portés par le même axe longitudinal (XX') du corps (2) et sont prévus de diamètres différents.

10. Ancre de suture suivant la revendication 1, **caractérisée en ce que** les butées (14) présentent une longueur qui dépend de la déformation que l'on désire obtenir des branches de fixation (6, 7).

11. Ancre de suture suivant la revendication 2, **caractérisée en ce que** la pointe à profil conique (8) présente une face (15) qui est disposée perpendiculairement à l'axe (XX').

12. Ancre de suture suivant la revendication 3, **caractérisée en ce que** l'espace oblong (20) des moyens de fixation (4) est positionné de manière que sa direction de plus grande longueur soit perpendiculaire à l'axe longitudinal (XX') du corps (2).

13. Ancre de suture suivant la revendication 3, **caractérisée en ce que** la tête (3) comporte une partie cylindrique (22) pourvue sur sa périphérie externe d'une collerette circulaire (24) formant une butée d'appui du corps creux (2) contre la paroi externe de l'os (17).

14. Ancre de suture suivant la revendication 4, **caractérisée en ce que** la tête (3') présente une partie cylindrique (22) et une partie filetée d'un alésage interne (9) qui est disposée à proximité du disque (26).

## Patentansprüche

1. Anker für chirurgischen Nähfaden, der die Befestigung von Weichgeweben (16) an einem Knochen (17) gestattet, umfassend einen Hohlkörper (2), der einerseits an einem seiner Enden einen Kopf (3, 3'), der mit Mitteln zur Befestigung (4) der Weichgewebe (16) am Knochen (17) mittels Hindurchleiten mindestens eines chirurgischen Nähfadens (5) versehen ist, und andererseits Spreizmittel zur Befestigung des Ankers für chirurgischen Nähfaden im Knochen (17) umfasst, wobei die Spreizmittel aus mindestens zwei Befestigungsästen (6, 7), die parallel zur Längsachse (XX' ) des Hohlkörpers vor der Verformung angebracht sind, und aus mindestens zwei Anschlägen (14) bestehen, die zwischen jedem Befestigungsast (6, 7) eingebracht sind und die plastische Verformung der Letzteren bei Anwendung einer externen Zugkraft (T) zur Befestigung des Ankers für chirurgischen Nähfaden (1) im Knochen beschränken (17), damit die Verformung der Spreizmittel (6, 7) bei Anwendung einer anderen externen Schubkraft (P) wieder lösbar ist, was das Entnehmen des Ankers für chirurgischen Nähfaden (1) vom Knochen (17) gestattet.

2. Anker für chirurgischen Nähfaden nach Anspruch 1, **dadurch gekennzeichnet, dass** der Hohlkörper (2) an dem Ende, das dem Kopf (3, 3') gegenüberliegt, eine Spitze mit konischem Profil (8) umfasst.

3. Anker für chirurgischen Nähfaden nach Anspruch 1, **dadurch gekennzeichnet, dass** die Befestigungsmittel (4) des Kopfes (3) aus zwei gegenüberliegenden Anhängseln (18, 19) bestehen, die zur Längsachse (XX' ) des Hohlkörpers (2) vor der Verformung parallel sind, so dass sie einen länglichen Raum (20) umgeben, der von mindestens einem chirurgischen Nähfaden (5) durchlaufen wird, wobei dieser chirurgische Nähfaden (5) nach der Verformung der Anhängsel (18, 19) unter einer anderen Zugkraft an den Kopf (3) gepresst wird, so dass die Verbindung der Weichgewebe (16) mit dem Anker für chirurgischen Nähfaden (1) gestattet wird.

4. Anker für chirurgischen Nähfaden nach Anspruch 1, **dadurch gekennzeichnet, dass** die Befestigungsmittel (4) des Kopfes (3') aus einer Scheibe (26) bestehen, die rechtwinklig zur Längsachse (XX' ) des Hohlkörpers (2) angebracht ist, wobei die Scheibe derart gestaltet ist, dass sie die Weichgewebe (16) gegen den Knochen (17) klemmt.

5. Anker für chirurgischen Nähfaden nach Anspruch 2, **dadurch gekennzeichnet, dass** der Hohlkörper (2) an der Spitze mit konischem Profil (8) ein gewindetes Grundloch (10) besitzt, das dazu vorgesehen ist, eine Schraubenspindel (28) eines Hilfsmittels (27) aufzunehmen, um einerseits unter einer ersten Zugkraft (T) die Befestigungsäste (6, 7) in eine im Wesentlichen rechtwinklige Richtung zur Längsachse (XX' ) des Körpers und andererseits unter einer zweiten Zugkraft (T1, T2), die höher ist als die erste (T), die Befestigungsmittel (4) zu verformen.

6. Anker für chirurgischen Nähfaden nach Anspruch 1, **dadurch gekennzeichnet, dass** der Hohlkörper (2) in der Nähe des Kopfes (3, 3' ) eine gewindete interne Bohrung (9) besitzt, die dazu bestimmt ist, eine erste hohle Schraubenspindel (36) eines Hilfsmittels (35) aufzunehmen, während eine zweite Spindel (37), die mit der ersten Spindel (36) ineinander schiebbar ist, unterstützend in den Boden eines gewindeten Grundlochs (10) kommt, damit unter einer Schubkraft (P) die Befestigungsäste (6, 7) in eine im Wesentlichen längliche Position auseinander gefaltet werden, so dass der Anker für chirurgischen Nähfaden (1) vom Knochen (17) entnommen werden kann.

7. Anker für chirurgischen Nähfaden nach Anspruch 2, **dadurch gekennzeichnet, dass** die Befestigungsäste (6, 7) an einem zylindrischen Abschnitt (22) des Kopfes (3, 3') und an der Spitze mit konischem Profil (8) durch erste Faltungsstücke (11) befestigt sind, die zum Zentrum des Hohlkörpers (2) gerichtet sind.

8. Anker für chirurgischen Nähfaden nach Anspruch 6, **dadurch gekennzeichnet, dass** die Befestigungsäste (6, 7) jeweils in ihrer Umgebung ein zweites Faltungsstück (12, 13) besitzen, das in Bezug auf die ersten Faltungsstücke (11) derart umgekehrt ist, dass jeder Ast aus zwei identischen Segmenten (6a, 6b; 7a, 7b) besteht.

9. Anker für chirurgischen Nähfaden nach Anspruch 6, **dadurch gekennzeichnet, dass** die gewindete interne Bohrung (9) und das gewindete Grundloch (10) die gleiche Längsachse (XX' ) des Körpers (2) besitzen und mit unterschiedlichen Durchmessern vorgesehen sind.

10. Anker für chirurgischen Nähfaden nach Anspruch 1, **dadurch gekennzeichnet, dass** die Anschläge (14) eine Länge aufweisen, die von der Verformung der Befestigungsäste (6, 7) abhängt, die man erhalten möchte.

11. Anker für chirurgischen Nähfaden nach Anspruch 2, **dadurch gekennzeichnet, dass** die Spitze mit konischem Profil (8) eine Seite (15) aufweist, die senkrecht zur Achse (XX') angebracht ist.

12. Anker für chirurgischen Nähfaden nach Anspruch 3, **dadurch gekennzeichnet, dass** der längliche Raum (20) des Befestigungsmittels (4) derart positioniert ist, dass die Richtung seiner größten Längenausdehnung senkrecht zur Längsachse (XX' ) des Körpers (2) verläuft.

13. Anker für chirurgischen Nähfaden nach Anspruch 3, **dadurch gekennzeichnet, dass** der Kopf (.3) einen zylindrischen Teil (22) besitzt, der an seiner äußeren Peripherie mit einem ringförmigen Kragen (24) versehen ist, der einen unterstützenden Anschlag des Hohlkörpers (2) gegen die Außenwand des Knochens (17) bildet.

14. Anker für chirurgischen Nähfaden nach Anspruch 4, **dadurch gekennzeichnet, dass** der Kopf (3') einen ringförmigen Teil (22) und einen gewindeten Teil einer internen Bohrung (9) aufweist, der in der Nähe der Scheibe (26) angebracht ist.

## Claims

1. Suture anchor permitting fixation of soft tissues (16) against a bone (17), with a hollow body (2) comprising on the one hand, at one of its ends, a head (3, 3') provided with means (4) for fixing the soft tissues (16) against the bone (17) by passing at least one suture thread (5) through it, and, on the other hand, expansion means for fixing the suture anchor in the bone (17), said expansion means being formed by at least two fixation branches (6, 7) which are arranged parallel to the longitudinal axis (XX' ) of said hollow body before deformation, and at least two stops (14) interposed between each fixation branch (6, 7) and limiting the plastic deformation of these branches upon application of an external tractive force (T) for fixing the suture anchor (1) in the bone (17), so that the deformation of said expansion means (6, 7) is reversible upon application of another external force, a thrust force (P), permitting the withdrawal of said suture anchor (1) from the bone (17).

2. Suture anchor according to Claim 1, **characterized in that** the hollow body (2) comprises, at the end remote from the head (3, 3'), a conically profiled point (8).

3. Suture anchor according to Claim 1, **characterized in that** the fixation means (4) of the head (3) are formed by two opposite tongues (18, 19) parallel to the longitudinal axis (XX' ) of the hollow body (2) before deformation, in order to delimit an oblong space (20) through which at least one suture thread (5) passes, said suture thread (5) being crimped on the head (3) after deformation of the tongues (18, 19) under another tractive force to permit ligation of the soft tissues (16) on the suture anchor (1).

4. Suture anchor according to Claim 1, **characterized in that** the fixation means (4) of the head (3) are formed by a disc (26) which is arranged perpendicular to the longitudinal axis (XX' ) of the hollow body (2), said disc being configured so as to block the soft tissues (16) against the bone (17).

5. Suture anchor according to Claim 2, **characterized in that** the hollow body (2) comprises, in the conically profiled point (8), a threaded blind hole (10) intended to receive a threaded rod (28) of an ancillary component (27) in order to deform, on the one hand under a first tractive force (T), the fixation branches (6, 7) in a direction substantially perpendicular to the longitudinal axis (XX' ) of said body and, on the other hand, to deform the fixation means (4) under a second tractive force (T1, T2) greater than the first (T).

6. Suture anchor according to Claim 1, **characterized in that** the hollow body (2) comprises, near the head (3, 3'), a threaded internal bore (9) which is intended to receive a first hollow threaded rod (36) of an ancillary component (35), while a second rod (37) sliding in the first rod (36) comes to bear in the bottom of a threaded blind hole (10) so that, under a thrust force (P), the fixation branches (6, 7) are folded out into a substantially elongate position in order to make it possible to withdraw the suture anchor (1) from the bone (17).

7. Suture anchor according to Claim 2, **characterized in that** the fixation branches (6, 7) are connected to a cylindrical part (22) of the head (3, 3') and to the conically profiled point (8) by first bend initiators (11) oriented in the direction of the centre of the hollow body (2).

8. Suture anchor according to Claim 6, **characterized in that** the fixation branches (6, 7) each have, at their centre, a second bend initiator (12, 13), which is inverted in relation to the first bend initiators (11) in such a way that each branch is made up of two identical segments (6a, 6b; 7a, 7b).

9. Suture anchor according to Claim 6, **characterized in that** the threaded internal bore (9) and the threaded blind hole (10) are carried by the same longitudinal axis (XX') of the body (2) and are provided with different diameters.

10. Suture anchor according to Claim 1, **characterized in that** the stops (14) have a length which depends on the desired deformation of the fixation branches (6, 7).

11. Suture anchor according to Claim 2, **characterized in that** the conically profiled point (8) has a face (15) arranged perpendicular to the axis (XX').

12. Suture anchor according to Claim 3, **characterized in that** the oblong space (20) of the fixation means (4) is positioned in such a way that its direction of greatest length is perpendicular to the longitudinal axis (XX' ) of the body (2).

13. Suture anchor according to Claim 3, **characterized in that** the head (3) comprises a cylindrical part (22) provided on its outer periphery with a circular flange (24) forming a supporting stop for the hollow body (2) against the outer wall of the bone (17).

14. Suture anchor according to Claim 4, **characterized in that** the head (3') has a cylindrical part (22) and a threaded part of an internal bore (9) arranged near the disc (26).
